# EUROPEAN PATENT APPLICATION

(11) **EP 4 206 672 A1**
(43) Date of publication of application: **05.07.2023**
(21) Application number: 21866781.4
(22) Date of filing: 08.09.2021
(51) Int. Cl.: G01N 33/543

(54) **TEST KIT HAVING SPECIFICITY IMPROVED BY INHIBITING FALSE POSITIVES**

(30) Priority: 08.09.2020 JP 2020150743
(71) Applicant: Denka Company Limited, Tokyo 103-8338 (JP)
(72) Inventor: SHINOHARA Yuki, Gosen-shi, Niigata 959-1695 (JP); SATO Ai, Gosen-shi, Niigata 959-1695 (JP)
(74) Representative: Clarenbach, Carl-Philipp
(86) International application number: PCT/JP2021/032953
(87) International publication number: WO 2022/054822

(57) **Abstract**

This invention relates to a specimen-extracting solution containing a component capable of strongly suppressing false positives, which could not be suppressed by conventional techniques, a method for extracting specimens, and a test reagent using such specimen-extracting solution and such method, when detecting virus, bacteria, target protein, or other antigens from specimens derived from body fluids, such as nasal swab specimens, nasal aspirate specimens, nasal wash specimens, nasal secretion specimens collected by nose blowing, pharyngeal swab specimens, saliva specimens, fecal specimens, serum specimens, plasma specimens, and urine specimens, with the use of a detection reagent utilizing the antigen-antibody reactions or the reactions between substances interactive with each other. The specimen-extracting solution as a constitutional unit of the test reagent or a member brought into contact with the specimens in a step performed before the detection reaction or a step performed simultaneously with the detection reaction is supplemented with a water-soluble compound comprising a phenyl, benzyl, tolyl, or xylyl group and, bound thereto, at least a carboxyl group, a functional group comprising methylated/ethylated atoms thereof, or a hydroxyl group.

## Description

### Technical Field

The present invention relates to a technique that can strongly suppress false positives, which could not be sufficiently suppressed according to conventional techniques, with the use of a compound having a particular chemical structure as a specimen-extracting solution or a member brought into contact with specimens in a step performed before the detection reaction or a step performed simultaneously with the detection reaction, when detecting target substances, such as viruses, bacteria, and target proteins, from specimens derived from body fluids, such as nasal swab specimens, nasal aspirate specimens, nasal wash specimens, nasal secretion specimens collected by nose blowing, pharyngeal swab specimens, saliva specimens, fecal specimens, serum specimens, plasma specimens, and urine specimens, using test reagents utilizing the antigen-antibody reactions or the binding reactions between substances interactive with each other.

### Background Art

In recent years, development of a wide variety of test reagents or kits intended to detect infection with pathogens, such as viruses or bacteria, the pregnancy status, or the like utilizing the antigen-antibody reactions or the binding reactions between substances interactive with each other has been in progress. It is important for such test reagents to perform a step of pretreatment for creating conditions suitable for the detection reaction after the specimens are collected from patients in order to obtain accurate results. In particular, many rapid test reagents do not require the use of any special equipment and can be operated in a simple and cost-effective manner. In addition, such rapid test reagents are extensively used in hospitals or clinics for general patients, in addition to large hospitals or medical testing centers, and are often used by users other than medical testing professionals. Accordingly, it is very critical that reagents can provide high test accuracy. Examples of rapid test reagents that are commercially available at present include rapid test reagents for detecting pathogen infection and rapid test reagents for pregnancy diagnosis. Such test reagents are often used in medical institutions where patients visit for the first time, the specimens collected from patients can be tested for infection or pregnancy on site, and treatment can be provided at an early stage. Thus, importance of rapid test reagents is increasing in clinical settings. As the use of rapid test reagents increases, reagent performance, such as the test results and the test accuracy with higher reproducibility, is desired by users.

At present, representative techniques involving the use of reagents for rapid testing, such as an immunoassay technique utilizing the antigen-antibody reactions, and, in particular, an immunochromatography method, are generally known. According to an immunochromatography method, a complex of a capture substance specifically binding to a target substance and a label specifically binding to a target substance is formed on a membrane, and the label is detected/quantified to detect (measure or quantify) the target substance. An immunochromatography method uses a simple assay device and is excellent in terms of the cost, and, thus, such technique has been extensively used for detecting a wide variety of target substances.

An embodiment of the immunochromatography method involves the use of a test device comprising a detection unit composed of, for example, a nitrocellulose membrane strip and a capture antibody specifically binding to a target substance immobilized thereon and a label unit composed of a label specifically binding to the target substance. A specimen sample containing the target substance is applied dropwise to the test device, a target substance-label complex is formed and allowed to develop, and the complex is captured in the detection unit to detect or quantify the label.

In recent years, reagents used for clinical diagnosis involving immunochromatography methods that can provide more reliable results of diagnosis have been desired in clinical settings, and a further improvement is desired for reagent reliability. Test reagents with high reliability are high in sensitivity and specificity and such reagents are less likely to cause erroneous judgment. Concerning specificity, in particular, the design of reagents to deal with the diversity of specimen components derived from patients with different backgrounds has been an issue of concern. Accordingly, it is very critical to more effectively resolve a problem of non-specific reactions in a rapid testing method. In this respect, it has been reported that specificity can be improved to some extent by bringing basic amino acids such as arginine and lysine, inorganic salts, glycine ethyl ester, a surfactant, animal-derived immunoglobulin, or the like into contact with specimens (Patent Documents 1, 2, and 3). However, effects thereof are limited and some non-specific reactions remain insuppressible. Therefore, a technique that can improve specificity more effectively while refraining from lowering sensitivity is awaited.

### Prior Art Documents

### Patent Documents

Patent Document 1: JP 2003-279577 A
Patent Document 2: JP 2005-24323 A
Patent Document 3: JP 2004-301684 A

### Summary of the Invention

### Objects to Be Attained by the Invention

When detecting target substances, such as viruses, bacteria, and target proteins, from specimens derived from body fluids, such as nasal swab specimens, nasal aspirate specimens, nasal wash specimens, nasal secretion specimens collected by nose blowing, pharyngeal swab specimens, saliva specimens, fecal specimens, serum specimens, plasma specimens, or urine samples, with the use of detection reagents utilizing the antigen-antibody reactions or the binding reactions between substances interactive with each other, false positives and false negatives, which could not be sufficiently suppressed according to conventional techniques, still disturb accurate diagnosis. The present invention provides a test reagent involving the use of a specimen-extracting solution containing a component that can strongly suppress false positives or a method of extracting specimens without lowering sensitivity.

### Means for Attaining the Objects

The present inventors have conducted concentrated studies in order to find a method that can more strongly suppress non-specific reactions occurring when specimens derived from body fluids, such as nasal swab specimens, nasal aspirate specimens, nasal wash specimens, nasal secretion specimens collected by nose blowing, pharyngeal swab specimens, saliva specimens, fecal specimens, serum specimens, plasma specimens, and urine specimens, are used as test samples. As a result, they discovered a component that would suppress non-specific reactions more remarkably than conventional techniques. They also discovered that false positives, which had been detected according to conventional techniques, could be suppressed with the addition of the discovered component to a specimen-extracting solution or members brought into contact with the specimen in a step performed before the detection reaction or a step performed simultaneously with the detection reaction. This has led to the completion of the present invention.

Specifically, the present invention has the following features.
[1] A test reagent for detecting a target substance in a specimen utilizing the antigen-antibody reactions or the binding reactions between substances interactive with each other, which comprises a specimen-extracting solution containing a non-specific-reaction-suppressing component for suppressing false positives, which is a water-soluble compound with a molecular weight of 6,000 Da or lower containing a phenyl group, a benzyl group, a tolyl group, or a xylyl group, further containing a carboxyl group, a methoxycarboxyl group, or an ethoxycarboxyl group, and optionally containing a hydroxyl group.
[2] The test reagent according to [1], which is a test reagent for immunochromatography or a test device for immunochromatography comprising a specimen-extracting solution containing a non-specific-reaction-suppressing component for suppressing false positives, which is a water-soluble compound with a molecular weight of 6,000 Da or lower containing a phenyl group, a benzyl group, a tolyl group, or a xylyl group, further containing a carboxyl group, a methoxycarboxyl group, or an ethoxycarboxyl group, and optionally containing a hydroxyl group.
[3] The test reagent according to [1] or [2], which is a test device for immunochromatography comprising a site impregnated with a specimen-extracting solution containing a non-specific-reaction-suppressing component for suppressing false positives, which is a water-soluble compound with a molecular weight of 6,000 Da or lower containing a phenyl group, a benzyl group, a tolyl group, or a xylyl group, further containing a carboxyl group, a methoxycarboxyl group, or an ethoxycarboxyl group, and optionally containing a hydroxyl group.
[4] The test reagent according to any of [1] to [3], wherein the specimen-extracting solution contains 0.1 to 10 (w/v)% of a non-specific-reaction-suppressing component for suppressing false positives.
[5] The test reagent according to any of [1] to [4], which is a compound or tryptophan represented by any of Formulae (I) to (V) below: 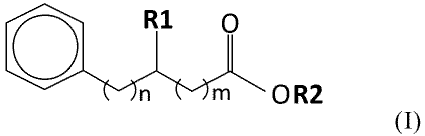 in Formula (I), R1 represents H, OH, =O, NH₂, COOH, NH-CO-CNH₂-C-COOH, or CH₃; R2 represents H, CH₃, C₂H₅, Li, Na, K, Rb, Cs, or Fr; n is 0 or 1; and m is 0, 1, 2, 3, or 4; 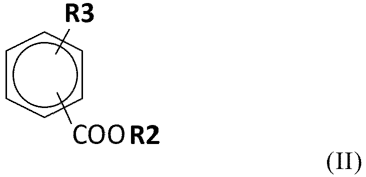 in Formula (II), R2 represents H, CH₃, C₂H₅, Li, Na, K, Rb, Cs, or Fr; R3 represents COOH, COOLi, COONa, COOK, COORb, COOCs, COOFr, COOCH₃, COOC₂H₅, OCOH, or CH₃; and R3 and COOR2 are each in the ortho-, meta-, or para-position in a benzene ring; 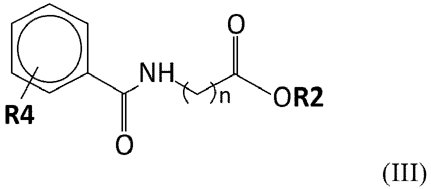 in Formula (III), R2 represents H, CH₃, C₂H₅, Li, Na, K, Rb, Cs, or Fr; R4 represents H or CH₃; and n is 0 or 1; 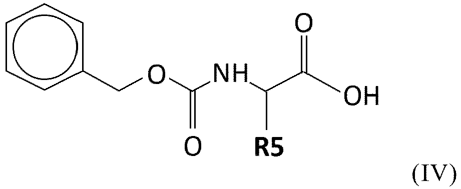 in Formula (IV), R5 is a side chain of glycine, alanine, valine, leucine, isoleucine, serine, threonine, cysteine, methionine, asparagine, glutamine, phenylalanine, tyrosine, tryptophan, glutamic acid, aspartic acid, arginine, lysine, or histidine; and 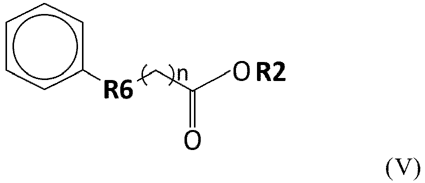 in Formula (V), R2 represents H, CH₃, C₂H₅, Li, Na, K, Rb, Cs, or Fr; R6 represents NH or O; and n is 0, 1, 2, 3, or 4.
[6] The test reagent according to any of [1] to [5], wherein the water-soluble compound with a molecular weight of 6,000 Da or lower containing a phenyl group, a benzyl group, a tolyl group, or a xylyl group, further containing a carboxyl group, a methoxycarboxyl group, or an ethoxycarboxyl group, and optionally containing a hydroxyl group is a compound selected from the group consisting of aspartame, phenylalanine, phenylalanine methyl ester, mandelic acid, 2-phenylpropionic acid, 3-phenylpropionic acid, phenylglycine, phenylglycine methyl ester, phenylglycine ethyl ester, phenyllactic acid, phenylpyruvic acid, benzoic acid, phthalic acid, acetylsalicylic acid, hippuric acid, N-toluoylglycine, N-carbobenzyloxyamino acid, N-phenylglycine, phenoxyacetic acid, tryptophan, metal salts of such compounds, and optical isomers, geometric isomers, constitutional isomers, stereoisomers, and positional isomers of such compounds.
[7] The test reagent according to any of [1] to [6], wherein the specimen-extracting solution is further containing an amino acid or an amino acid derivative selected from the group consisting of arginine, lysine, arginine ethyl ester, arginine methyl ester, glycine ethyl ester, glycine methyl ester, and optical isomers, geometric isomers, constitutional isomers, and stereoisomers of such compounds.
[8] The test reagent according to any of [1] to [7], wherein the specimen-extracting solution is further containing a halide selected from the group consisting of lithium chloride, sodium chloride, potassium chloride, sodium bromide, potassium bromide, sodium iodide, and potassium iodide.
[9] A method for detecting a target substance selected from the group consisting of a virus antigen, a bacterial antigen, and a protein antigen in a specimen selected from the group consisting of a pharyngeal swab specimen, a nasal swab specimen, a nasal aspirate specimen, a pharyngeal wash specimen, a nasal wash specimen, a nasal secretion specimen collected by nose blowing, a saliva specimen, a serum specimen, a plasma specimen, a whole blood specimen, a fecal specimen, a fecal suspension specimen, and an urine specimen utilizing the antigen-antibody reactions or the reactions between substances interactive with each other in a specimen-extracting solution while suppressing the false positives, wherein the specimen is brought into contact with a non-specific-reaction-suppressing component for suppressing false positives, which is a water-soluble compound with a molecular weight of 6,000 Da or lower containing a phenyl group, a benzyl group, a tolyl group, or a xylyl group, further containing a carboxyl group, a methoxycarboxyl group, or an ethoxycarboxyl group, and optionally containing a hydroxyl group, in advance.
[10] The method according to [9], wherein the method for detecting a target substance is an immunochromatography method comprising introducing a specimen into a specimen-extracting solution containing a non-specific-reaction-suppressing component for suppressing false positives, which is a water-soluble compound with a molecular weight of 6,000 Da or lower containing a phenyl group, a benzyl group, a tolyl group, or a xylyl group, further containing a carboxyl group, a methoxycarboxyl group, or an ethoxycarboxyl group, and optionally containing a hydroxyl group, and applying the specimen-extracting solution to a test device for immunochromatography.
[11] The method according to [9], wherein the method for detecting a target substance is an immunochromatography method comprising applying a specimen to a test device for immunochromatography comprising a site impregnated with a specimen-extracting solution containing a non-specific-reaction-suppressing component for suppressing false positives, which is a water-soluble compound with a molecular weight of 6,000 Da or lower containing a phenyl group, a benzyl group, a tolyl group, or a xylyl group, further containing a carboxyl group, a methoxycarboxyl group, or an ethoxycarboxyl group, and optionally containing a hydroxyl group.
[12] The method according to any of [9] to [11], wherein the specimen-extracting solution contains 0.1 to 10 (w/v)% of a non-specific-reaction-suppressing component for suppressing false positives.
[13] The method according to any of [9] to [12], wherein the water-soluble compound with a molecular weight of 6,000 Da or lower containing a phenyl group, a benzyl group, a tolyl group, or a xylyl group, further containing a carboxyl group, a methoxycarboxyl group, or an ethoxycarboxyl group, and optionally containing a hydroxyl group is a compound or tryptophan represented by any of Formulae (I) to (V) below: 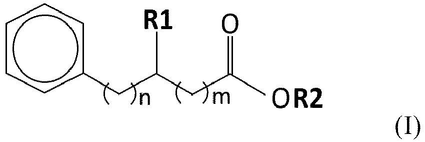 in Formula (I), R1 represents H, OH, =O, NH₂, COOH, NH-CO-CNH₂-C-COOH, or CH₃; R2 represents H, CH₃, C₂H₅, Li, Na, K, Rb, Cs, or Fr; n is 0 or 1; and m is 0, 1, 2, 3, or 4; 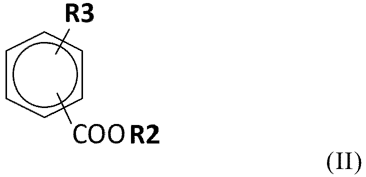 in Formula (II), R2 represents H, CH₃, C₂H₅, Li, Na, K, Rb, Cs, or Fr; R3 represents COOH, COOLi, COONa, COOK, COORb, COOCs, COOFr, COOCH₃, COOC₂H₅, OCOH, or CH₃; and R3 and COOR2 are each in the ortho-, meta-, or para-position in a benzene ring; 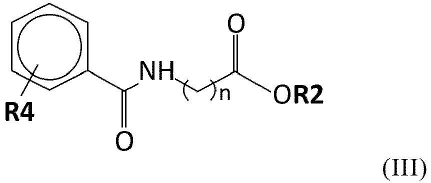 in Formula (III), R2 represents H, CH₃, C₂H₅, Li, Na, K, Rb, Cs, or Fr; R4 represents H or CH₃; and n is 0 or 1; 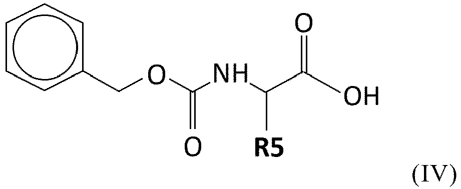 in Formula (IV), R5 is a side chain of glycine, alanine, valine, leucine, isoleucine, serine, threonine, cysteine, methionine, asparagine, glutamine, phenylalanine, tyrosine, tryptophan, glutamic acid, aspartic acid, arginine, lysine, or histidine; and 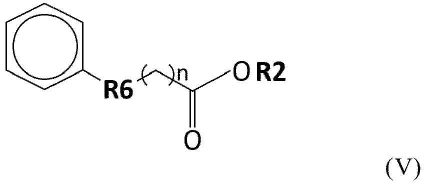 in Formula (V), R2 represents H, CH₃, C₂H₅, Li, Na, K, Rb, Cs, or Fr; R6 represents NH or O; and n is 0, 1, 2, 3, or 4.
[14] The method according to any of [9] to [13], wherein the water-soluble compound with a molecular weight of 6,000 Da or lower containing a phenyl group, a benzyl group, a tolyl group, or a xylyl group, further containing a carboxyl group, a methoxycarboxyl group, or an ethoxycarboxyl group, and optionally containing a hydroxyl group is a compound selected from the group consisting of aspartame, phenylalanine, phenylalanine methyl ester, mandelic acid, 2-phenylpropionic acid, 3-phenylpropionic acid, phenylglycine, phenylglycine methyl ester, phenylglycine ethyl ester, phenyllactic acid, phenylpyruvic acid, benzoic acid, phthalic acid, acetylsalicylic acid, hippuric acid, N-toluoylglycine, N-carbobenzyloxyamino acid, N-phenylglycine, phenoxyacetic acid, tryptophan, metal salts of such compounds, and optical isomers, geometric isomers, constitutional isomers, stereoisomers, and positional isomers of such compounds.
[15] The method according to any of [9] to [14], wherein the specimen-extracting solution is further supplemented with an amino acid or an amino acid derivative selected from the group consisting of arginine, lysine, arginine ethyl ester, arginine methyl ester, glycine ethyl ester, glycine methyl ester, and optical isomers, geometric isomers, constitutional isomers, and stereoisomers of such compounds.
[16] The method according to any of [9] to [15], wherein the specimen-extracting solution is further supplemented with a halide selected from the group consisting of lithium chloride, sodium chloride, potassium chloride, sodium bromide, potassium bromide, sodium iodide, and potassium iodide.

This description includes part or all of the content as disclosed in the description and/or drawings of Japanese Patent Application No. 2020-150743, which is a priority document of the present application.

### Effects of the Invention

The present invention can provide a test reagent for detecting particular viruses, bacteria, proteins, low-molecular-weight compounds, and the like from specimens derived from body fluids, such as nasal swab specimens, nasal aspirate specimens, nasal wash specimens, nasal secretion specimens collected by nose blowing, pharyngeal swab specimens, saliva specimens, fecal specimens, serum specimens, plasma specimens, and urine specimens, utilizing the antigen-antibody reactions or the binding reactions between substances interactive with each other, which can more strongly suppress false positives occurring as a result of contamination of specimens and achieve high reproducibility and high test accuracy. In addition, such reagent can prevent erroneous clinical diagnosis caused by non-specific reactions and thus is beneficial for both patients and users, such as physicians, laboratory technicians, and nurses.

### Brief Description of the Drawing

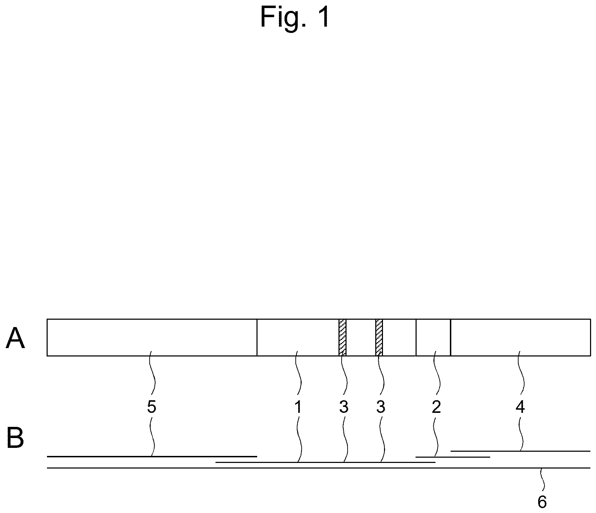

Fig. 1 shows a structure of a test device used in the present invention. Embodiments of the Invention

Hereafter, the present invention is described in detail.

The present invention relates to a method of suppressing false positives and preventing signal intensity; i.e., sensitivity, from lowering by bringing a compound into contact with a specimen when detecting a target substance in the specimen with the use of a detection reagent utilizing the antigen-antibody reactions or the binding reactions between substances interactive with each other.

In the present invention, an antibody includes an antigen-binding fragment of an antibody.

### (Specimens)

Specimens to be used herein are not limited. Examples of specimens include pharyngeal swab liquid, nasal swab liquid, nasal aspirate, pharyngeal wash liquid, nasal wash liquid, nasal secretion collected by nose blowing, saliva, serum, plasma, whole blood, fecal suspension, urine, and culture solution. Such specimens are referred to as a pharyngeal swab specimen, a nasal swab specimen, a nasal aspirate specimen, a pharyngeal wash specimen, a nasal wash specimen, a nasal secretion specimen collected by nose blowing, a saliva specimen, a serum specimen, a plasma specimen, a whole blood specimen, a fecal suspension specimen, an urine specimen, and a culture solution specimen. Such specimens can be diluted with a buffer and used in that state, or the specimens can be used without being diluted.

### (Target substances)

Target substances to be detected may be any substances without particular limitation. Specific examples include: viral antigens, such as influenza virus, adenovirus, RS (respiratory syncytial) virus, human metapneumoviruses (hMPV), hepatitis A virus (HAV), hepatitis B virus (HBV), human immunodeficiency virus (HIV), norovirus, and coronavirus, such as SARS-CoV, MERS-CoV, and SARS-CoV2, antigens; bacterial antigens, such as methicillin-resistant Staphylococcus aureus (MRSA), Streptococcus group A, Streptococcus group B, and Legionella antigens; toxins produced by bacteria; Mycoplasma antigens; Chlamydia antigens, such as Chlamydia trachomatis; protozoan antigens; fungal antigens; hormones, such as human chorionic gonadotropin; proteins, such as C-reactive protein, myoglobin, cardiac troponin, and procalcitonin; various tumor markers; antigens, such as agricultural chemical and environmental hormone antigens; and antibodies reacting with the bacteria and the viruses described above.

### (Collection of specimens)

Methods for collecting specimens are not limited. Examples of methods for collecting specimens derived from body fluids, such as pharyngeal swab specimens, nasal swab specimens, nasal aspirate specimens, pharyngeal wash specimens, nasal secretion specimens collected by nose blowing, saliva specimens, serum specimens, plasma specimens, whole blood specimens, fecal specimens, fecal suspension specimens, urine specimens, and culture solution specimens, and specimens derived from excretion products include a method for collecting specimens using a specimen-collecting tool such as cotton swabs, a method for collecting specimens by suction using a suction apparatus, and a method for collecting specimens using a blood collection tube.

### (Contact between the non-specific-reaction-suppressing component for suppressing false positives and the collected specimens)

In the method of the present invention, the specimens are brought into contact with the non-specific-reaction-suppressing component for suppressing false positives. Thus, false positives can be suppressed when assaying the specimens. When the specimens are brought into contact with the non-specific-reaction-suppressing component for suppressing false positives, the target substance is brought into contact with the non-specific-reaction-suppressing component for suppressing false positives. In the method of the present invention, accordingly, the situation in which the specimens are brought into contact with the non-specific-reaction-suppressing component for suppressing false positives may be regarded as the situation in which the target substance is brought into contact with the non-specific-reaction-suppressing component for suppressing false positives. The situation in which the specimens are brought into contact with the non-specific-reaction-suppressing component for suppressing false positives may be regarded as the treatment of the specimens with the non-specific-reaction-suppressing component for suppressing false positives.

The specimen-extracting solution is a liquid that suspends the target substance in the specimens to facilitate assays. For example, it is not necessary to extract a specific target substance from cells by dissolution, and the specimen-extracting solution can also be simply referred to as a specimen treatment solution, a specimen diluent, or a specimen suspension.

In the present invention, it is necessary to bring the specimens into contact with the non-specific-reaction-suppressing component for suppressing false positives before the test. The time "before the test" is before the reaction between the target substance in the specimens and the antibody or antigen reacting therewith takes place or before the reaction between the target substance in the specimens and the substances interactive therewith takes place. In the reaction between the target substance in the specimens and the antibody or antigen, the target substance binds to the antibody or antigen. In the reaction between the target substance in the specimens and the substances interactive therewith, the target substance binds to the substances interactive therewith.

Examples of methods for bringing the non-specific-reaction-suppressing component for suppressing false positives into contact with specimens in the present invention include: a method in which specimens are introduced into a solution containing the non-specific-reaction-suppressing component for suppressing false positives to be mixed with and brought into contact with the non-specific-reaction-suppressing component for suppressing false positives; and a method in which a test device used for assays is impregnated with a specimen-extracting solution containing the non-specific-reaction-suppressing component for suppressing false positives, the specimens are applied to the test device used for assays, and the specimens are brought into contact with the non-specific-reaction-suppressing component for suppressing false positives.

A specific example of a method in which specimens are introduced into a solution containing the non-specific-reaction-suppressing component for suppressing false positives to be mixed with and brought into contact with the non-specific-reaction-suppressing component for suppressing false positives is a method in which the non-specific-reaction-suppressing component for suppressing false positives is contained in a specimen-extracting solution in which the collected specimens are to be suspended and dispersed, and, when the specimens are added to and mixed with the specimen-extracting solution, the specimens are brought into contact with the non-specific-reaction-suppressing component for suppressing false positives. When a specimen-extracting solution containing the non-specific-reaction-suppressing component for suppressing false positives is used, for example, nasal swab specimens are collected using cotton swabs, the cotton swabs impregnated with the collected specimens are introduced into the specimen-extracting solution to suspend, disperse, and extract the specimens, and the specimens can be brought into contact with the non-specific-reaction-suppressing component for suppressing false positives.

A specific example of a method in which a specimen-extracting solution containing the non-specific-reaction-suppressing component for suppressing false positives is contained in a test device used for assays is impregnated with, the specimens are applied to the test device, and the specimens are brought into contact with the non-specific-reaction-suppressing component for suppressing false positives is a method in which a specimen-extracting solution containing the non-specific-reaction-suppressing component for suppressing false positives is contained in a fibrous or porous substrate such as a pad or filtration filter comprising unwoven fabric, woven fabric, or sponge of the test device by being impregnated or coated and so on, and, when the collected specimens are applied to the test device, the specimens are brought into contact with the non-specific-reaction-suppressing component for suppressing false positives, which has been contained in the fibrous or porous substrate. An example of such test device is the device for immunochromatography described below.

Materials constituting the porous substrate of the test device are not limited, and examples thereof include pulp, cotton, wool, polyester, polypropylene, nylon, acrylic glass fiber, and nitrocellulose. When a specimen-extracting solution containing the non-specific-reaction-suppressing component for suppressing false positives is contained in the test device used for assays and the specimens are applied to the test device and brought into contact with the non-specific-reaction-suppressing component for suppressing false positives, for example, a porous substrate may be impregnated with the specimen-extracting solution containing the non-specific-reaction-suppressing component for suppressing false positives, the porous substrate may be dried, and the specimens may then be brought into contact with the porous substrate on the test device in the step performed before or simultaneously with the reaction for detecting the target substance. When the specimens are applied to the test device, for example, the specimens spread on the test device to reach a site where the reaction takes place, and the antibody-antigen reaction or other reaction takes place. In a site before the site where the reaction takes place on the test device, a porous substrate impregnated with a specimen-extracting solution containing a non-specific-reaction-suppressing component for suppressing false positives is provided. Thus, the specimens are brought into contact with the non-specific-reaction-suppressing component for suppressing false positives before the reaction. For example, a filtration filter may be used as a porous substrate, and a filtration filter containing a specimen-extracting solution containing a non-specific-reaction-suppressing component for suppressing false positives may be provided in a site where the specimens are applied. When the nasal swab specimens are used, the nasal swab specimens are collected using cotton swabs, the cotton swabs impregnated with the collected specimens are introduced into the specimen-extracting solution of any composition that does not contain the non-specific-reaction-suppressing component for suppressing false positives to disperse and dissolve the specimens, and the constitutional member of the test device; i.e., the filtration filter impregnated with the non-specific-reaction-suppressing component for suppressing false positives, is impregnated with the specimen-extracting solution. Thus, the specimens can be brought into contact with the non-specific-reaction-suppressing component for suppressing false positives.

### (Non-specific-reaction-suppressing component for suppressing false positives and concentration thereof)

The term "non-specific-reaction-suppressing component for suppressing false positives" used in the present invention refers to a water-soluble compound with a molecular weight of 6,000 Da or lower containing a phenyl group, a benzyl group, a tolyl group, or a xylyl group, further containing a carboxyl group, a methoxycarboxyl group, or an ethoxycarboxyl group, and optionally containing a hydroxyl group. Also, an optical isomer, a geometric isomer, a constitutional isomer, a stereoisomer, and a positional isomer containing metal salts of such compounds are within the scope of such component.

Such compound is a compound or tryptophan represented by any of Formulae (I) to (V) below. 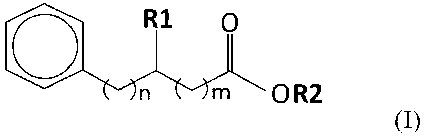 in Formula (I), R1 represents H, OH, =O, NH₂, COOH, NH-CO-CNH₂-C-COOH, or CH₃; R2 represents H, CH₃, C₂H₅, Li, Na, K, Rb, Cs, or Fr; n is 0 or 1; and m is 0, 1, 2, 3, or 4; 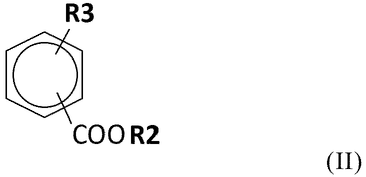 in Formula (II), R2 represents H, CH₃, C₂H₅, Li, Na, K, Rb, Cs, or Fr; R3 represents COOH, COOLi, COONa, COOK, COORb, COOCs, COOFr, COOCH₃, COOC₂H₅, OCOH, or CH₃; and R3 and COOR2 are each in the ortho-, meta-, or para-position in a benzene ring; 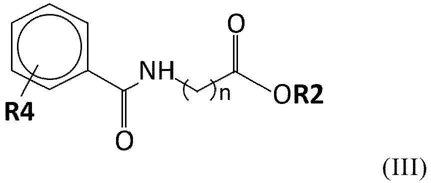 in Formula (III), R2 represents H, CH₃, C₂H₅, Li, Na, K, Rb, Cs, or Fr; R4 represents H or CH₃; and n is 0 or 1; 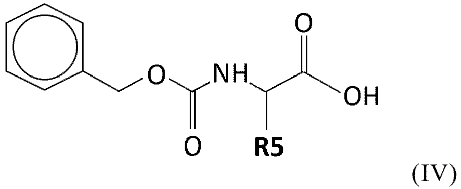 in Formula (IV), R5 is a side chain of glycine, alanine, valine, leucine, isoleucine, serine, threonine, cysteine, methionine, asparagine, glutamine, phenylalanine, tyrosine, tryptophan, glutamic acid, aspartic acid, arginine, lysine, or histidine; and 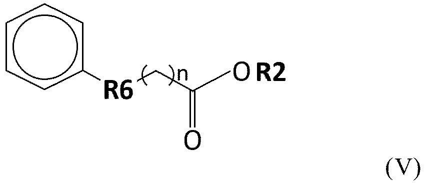 in Formula (V), R2 represents H, CH₃, C₂H₅, Li, Na, K, Rb, Cs, or Fr; R6 represents NH or O; and n is 0, 1, 2, 3, or 4.

Examples of compounds include, but are not limited to, compounds selected from the group consisting of aspartame, phenylalanine, phenylalanine methyl ester (including hydrochloride), mandelic acid, 2-phenylpropionic acid, 3-phenylpropionic acid, phenylglycine, phenylglycine methyl ester, phenylglycine ethyl ester, phenyllactic acid, phenylpyruvic acid, benzoic acid, phthalic acid, acetylsalicylic acid, hippuric acid, N-toluoylglycine, N-carbobenzyloxyamino acid, N-phenylglycine, phenoxyacetic acid, tryptophan, and an optical isomer, a geometric isomer, a constitutional isomer, a stereoisomer, and a positional isomer containing metal salts of such compounds. In the present invention, aspartame, phenylalanine, phenylalanine methyl ester (including hydrochloride), mandelic acid, 2-phenylpropionic acid, 3-phenylpropionic acid, phenylglycine, phenylglycine methyl ester, phenylglycine ethyl ester, phenyllactic acid, phenylpyruvic acid, benzoic acid, phthalic acid, acetylsalicylic acid, hippuric acid, N-toluoylglycine, N-carbobenzyloxyamino acid, N-phenylglycine, phenoxyacetic acid, and tryptophan include metal salts thereof, and further include optical isomers, geometric isomers, constitutional isomers, stereoisomers, and positional isomers thereof. Examples of metal salts include Li salt, Na salt, K salt, Rb salt, Cs salt, and Fr salt.

Examples of compounds represented by Formula (I) include aspartame, phenylalanine, phenylalanine methyl ester, mandelic acid, 2-phenylpropionic acid, 3-phenylpropionic acid, phenylglycine, phenylglycine methyl ester, phenylglycine ethyl ester, phenyllactic acid, phenylpyruvic acid, and benzoic acid.

Examples of compounds represented by Formula (II) include phthalic acid and acetylsalicylic acid.

Examples of compounds represented by Formula (III) include hippuric acid and N-toluoylglycine.

An example of a compound represented by Formula (IV) is N-carbobenzyloxyamino acid.

Examples of compounds represented by Formula (V) include N-phenylglycine and phenoxyacetic acid.

Such non-specific-reaction-suppressing component for suppressing false positives is incorporated into a specimen-extracting solution or a member such as a filtration filter that is brought into contact with the specimens in the step performed before the detection reaction. The concentration is preferably 0.001 (w/v)% or higher, more preferably 0.1 (w/v)% or higher, and most preferably 1 (w/v)% or higher. A plurality of types of non-specific-reaction-suppressing components can be simultaneously used, and, in such a case, the concentration is preferably 0.001 (w/v)% or higher, more preferably 0.1 (w/v)% or higher, and most preferably 1 (w/v)% or higher. While the upper limit of the concentration is not necessarily determined, the upper limit can be, for example, 10 (w/v)% or 5 (w/v)%. When a specimen-extracting solution containing a non-specific-reaction-suppressing component for suppressing false positives is contained in a porous substrate such as a filtration filter , the non-specific-reaction-suppressing component for suppressing false positives may be contained in the porous substrateat the concentration described above.

### (Components other than non-specific-reaction-suppressing components for suppressing false positives)

A specimen-extracting solution or a solution to be infiltrated into a member, such as a porous substrate, which is brought into contact with the specimens in the step performed before the detection reaction used in the present invention may contain, in addition to non-specific-reaction-suppressing components for suppressing false positives, known substances that can reduce non-specific reactions, surfactants, pHbuffering components, various proteins, salts, and saccharides. Examples of substances that can reduce non-specific reactions include arginine, arginine ethyl ester, arginine methyl ester, glycine ethyl ester, glycine methyl ester, lysine, and various isomers of such compounds. Examples of surfactants include nonionic surfactants, such as polyethylene glycol mono-p-isooctylphenyl ether and polyoxyethylene sorbitan monolaurate, amphoteric surfactants, such as CHAPS and laurylamide sulfobetaine, anionic surfactants, such as sodium dodecyl sulfate, and cationic surfactants, such as dodecyltrimethylammonium chloride. The concentration of the surfactant in the specimen-extracting solution is preferably 0.5 to 5 (w/v)%, more preferably 1 to 3 (w/v)%, and still more preferably 1.5 to 2.5 (w/v)%.

Examples of buffering components include phosphate buffer, Tris buffer, and Good's buffer. Examples of protein components include BSA (bovine serum albumin), casein, gelatin, and IgG. Examples of salts include lithium chloride, sodium chloride, potassium chloride, sodium bromide, potassium bromide, sodium iodide, and potassium iodide.

### (Method of detection)

In the method of the present invention, detection is performed by a method utilizing the antigen-antibody reactions or the binding reactions between substances interactive with each other. Examples of combinations of substances interactive with each other include a combination of a ligand and a receptor, a combination of a receptor and a receptor, and a combination of biotin and avidin or streptavidin.

The methods of detection utilizing the antigen-antibody reactions or the reactions between substances interactive with each other are not particularly limited, and examples of such methods include immunochromatography, latex aggregation, immunoturbidimetry, nephelometric immunoassay, chemoluminescence enzyme-linked immunosorbent assay (CLEIA), enzyme immunoassay (EIA), and enzyme-linked immunosorbent assay (ELISA). The method of immunochromatography is particularly preferable. Such technique is often an immunological method utilizing the antigen-antibody reactions, but the reactions between substances interactive with each other may be utilized instead of the antigen-antibody reactions. Among such techniques, the sandwich method is preferable. In a typical sandwich method, a first substance binding to a target substance is immobilized on a specific carrier as a target substance-capturing substance, the target substance is allowed to bind to the target substance-capturing substance, a second substance binding to a target substance, which has been labeled, is allowed to bind to the target substance, so as to form a complex of "the first substance binding to the target substance-the target substance-the labeled second substance binding to the target substance" (a symbol "-" represents a bond), and signals emitted from the labeled substance are measured to assay the target substance. The first substance binding to the target substance and the second substance binding to the target substance may be the same substances. A target substance and a substance binding to the target substance may be an antigen and an antibody, an antibody and an antigen, or substances interactive with each other. A solid phase on which the first substance binding to the target substance is to be immobilized can be any substance on which a protein or other substances can be immobilized in accordance with a conventional technique. For example, any known substance, such as a porous thin film (membrane) having a capillary action, a particulate substance, a test tube, or a resin flat plate, can be selected. Examples of substances that label the second substance binding to the target substance that can be used include an enzyme, a radioisotope, a fluorescent substance, a luminescent substance, a colored particle, and a colloidal particle.

Among sandwich methods, an immunochromatography method, which is a lateral flow type immunoassay method using a membrane, is particularly preferable from the viewpoint of simplicity and speed of clinical examination.

Hereafter, a general immunochromatography method utilizing the antigen-antibody reactions is described. Fig. 1 shows a test device used for immunochromatography.

Fig. 1 A shows a top view and Fig. 1 B shows a cross-sectional view. The test device comprises a plastic plate 6, a nitrocellulose membrane 1, and various portions superposed in that order on top of the other. In a specific example shown in Fig. 1, the test device comprises the plastic plate 6, the nitrocellulose membrane 1 comprising two detection parts 3 formed of target substance-capturing substances such as antibodies, the absorption pad part 5 formed of a filter paper, the label part 2, and the sample application part 4 formed of a glass fiber filter superposed in that order on top of the other.

As shown in Fig. 1, an end region of the absorption pad part 5 overlaps with an end region of the nitrocellulose membrane 1, the other end region of the nitrocellulose membrane 1 overlaps with an end region of the label part 2, and the other end region of the label part 2 overlaps with an end region of the sample application part 4. Thus, a continuous lateral flow fluid channel is formed.

The label part 2 comprises a label composed of a target substance-capturing substance, such as an antibody, and a labeling substance chemically or physically bound thereto. Examples of labeling substances include gold colloidal particles, platinum colloidal particles, color latex particles, magnetic particles, enzymes, quantum dots, fluorescent dyes, and phosphors. The label part is formed of a porous substrate containing the above-mentioned label, and the substrate material can be a common material, such as glass fiber or unwoven fabric. The porous substrate impregnated with the label and dried is referred to as a "stabilized dry label pad." Specifically, the label part is a portion comprising the stabilized dry label pad, which includes a colored latex particle-labeled antibody binding to the target substance by the antigen-antibody reactions.

The detection part 3 is a site where antibodies binding to the target substance by the antigen-antibody reactions are immobilized in a line as capture substances.

Examples of members that are brought into contact with the specimens in a step performed before the detection reaction of the target substance or a step performed simultaneously with the detection reaction include 1, 2, and 4 described above. Any members that are brought into contact with the specimens in a step performed before the detection reaction of the target substance or a step performed simultaneously with the detection reaction may be used without particular limitation. When the specimens are applied to the sample application part 4, the specimens flow from the sample application part 4 toward the absorption pad part 5. When the flow from the sample application part 4 toward the absorption pad part 5 is expressed as a downward flow, members that are brought into contact with the specimens in a step performed before the detection reaction of the target substance or a step performed simultaneously with the detection reaction are located upstream of the site where the detection reaction takes place.

Subsequently, an immunoassay method using the test device of the present invention is described. At the outset, the specimen is suspended in a specimen-extracting solution to prepare a specimen sample from which the target substance is extracted. Subsequently, the specimen sample is applied dropwise to the sample application part 4 of the test device. The specimen sample containing the target substance is pregnated into the label part 2 while migrating in a horizontal direction on the membrane to dissolve the label and develop. If the target substance is present in the specimen sample, a target substance-label complex is formed. When the complex reaches the detection part 3, a capture antibody-target substance-label complex is formed on the line of the detection part 3. The presence of the complex may be detected on the basis of signals emitted from the labeled substance in the complex, so as to determine the presence or absence of the target substance in the specimens. Other components that were not involved in the reaction are absorbed by the absorption pad part 5. In the example shown in Fig. 1, there are two detection parts 3, which are intended to capture two types of target substance, such as the influenza A virus and the influenza B virus, respectively. By providing a plurality of such detection parts 3, a plurality of types of target substances can be simultaneously immunoassayed.

In the immunochromatography method described above, the non-specific-reaction-suppressing component for suppressing false positives may be contained in the specimen-extracting solution mixed with the specimens to extract the target substance , or the non-specific-reaction-suppressing component for suppressing false positives may be contained in the nitrocellulose membrane 1, the label part 2, and/or the sample application part 4 of the test device for immunochromatography.

The present invention includes a test reagent utilizing the antigen-antibody reactions or the binding reactions between substances interactive with each other. The test reagent may be the test device, or the test reagent may be a test kit comprising the test device and other reagents. The test reagent of the present invention includes, for example, a test kit comprising a test device for immunochromatography and a specimen-extracting solution containing a non-specific-reaction-suppressing component for suppressing false positives. Also, the test reagent of the present invention includes a test device for immunochromatography comprising a site comprising a specimen-extracting solution containing a non-specific-reaction-suppressing component for suppressing false positives.

### Examples

The present invention is described in greater detail with reference to the following examples, although the scope of the present invention is not limited to these examples.

In the examples below, examples in which non-specific reactions of the specimens were suppressed in the detection performed using the kit for immunochromatography comprising the specimen-extracting solution of the present invention intended to detect influenza virus, RS virus, adenovirus, and Mycoplasma are described.

### Detection of influenza virus antigen by immunochromatography

### 1. Preparation of anti-influenza virus monoclonal antibodies

### (1) Anti-influenza A virus NP (nucleoprotein) antibodies

BALB/c mice were immunized with the influenza A virus antigen and raised for a given period of time. The spleens were removed from the mice and fused to mouse myeloma cells (P3×63) in accordance with the method of Kohler et al. (Kohler et al., Nature, vol. 256, pp. 495-497, 1975). The resulting fused cells (hybridomas) were maintained in an incubator at 37°C, and cells were purified (monoclonalized) while observing the antibody activity of the supernatant by ELISA using a plate comprising the influenza A virus NP antigen immobilized thereon. The 2 resulting cell lines were administered intraperitoneally to the pristane-treated BALB/c mice, and the antibody-containing ascites fluids were collected approximately 2 weeks later. IgG was purified from the obtained ascites by affinity chromatography using protein A columns to obtain 2 types of purified anti-influenza A virus NP antibodies.

### (2) Anti-influenza B virus NP antibodies

With the use of the influenza B virus antigen, 2 types of purified anti-influenza B virus NP antibodies were obtained in the same manner as in (1).

### 2. Preparation of label pad

One type of the purified anti-influenza A virus NP antibodies and one type of the purified anti-influenza B virus NP antibodies were used. The anti-influenza A virus NP antibody was allowed to covalently bind to red latex particles, suspended in a suspension, and subjected to sonication to thoroughly disperse and suspend latex particles. Thus, an anti-influenza A latex suspension was prepared. Separately, an anti-influenza B latex suspension was prepared by allowing the anti-influenza B virus NP antibody to covalently bind to blue latex particles. The anti-influenza A latex suspension was mixed with the anti-influenza B latex suspension, the mixture was applied to a glass fiber with a size of 20 cm × 1 cm, and the resultant was thoroughly dried with warm air to prepare a label pad formed of a dried mixture.

### 3. Preparation of sample application pad

A glass fiber with a size of 2.0 cm × 20 cm was used.

### 4. Preparation of test device

A test device of the same constitution as shown in Fig. 1 was used. On a nitrocellulose membrane cut to a size of 2 cm × 20 cm and backed with an adhesive plastic plate, a solution of the anti-influenza A virus antibody (an antibody different from the above antibody) and a solution of the anti-influenza B virus antibody (an antibody different from the above antibody) were each applied in a coating amount of about 1 mm in width at sites 0.8 cm and 1.0 cm from the lower end across the width of 20 cm, followed by thorough drying with warm air to immobilize the antibodies (the detection units). Subsequently, a filter paper with a size of 3 cm × 20 cm was laid on the nitrocellulose membrane to overlap therewith by 5 mm from the upper end of the nitrocellulose membrane to provide an absorption pad partt. Further, the label pad was laid on the nitrocellulose membrane to overlap therewith by 2 mm from the lower end of the nitrocellulose membrane to provide a label part. Further, the sample application pad was laid on the label pad at a position 7 mm away from the upper end of the label pad to provide a sample application part. Subsequently, the resultant was cut into strips with a width of 5 mm using a knife to obtain an integrated test device.

### Detection of RS virus, adenovirus, Mycoplasma pneumoniae antigens by immuno chromatography

### 1. Preparation of anti-RS virus, anti-adenovirus, and anti-Mycoplasma pneumoniae monoclonal antibodies

BALB/c mice were immunized with the RS virus antigen, the adenovirus antigen, or the Mycoplasma pneumoniae antigen and raised for a given period of time. The spleens were removed from the mice and fused to mouse myeloma cells (P3×63) in accordance with the method of Kohler et al. (Kohler et al., Nature, vol. 256, pp. 495-497, 1975). The resulting fused cells (hybridomas) were maintained in an incubator at 37°C, and cells were purified (monoclonalized) while observing the antibody activity of the supernatant by ELISA using a plate each comprising the antigen immobilized thereon. The 2 resulting cell lines were administered intraperitoneally to the pristane-treated BALB/c mice, and the antibody-containing ascites fluids were collected approximately 2 weeks later. IgG was purified from the obtained ascites by affinity chromatography using protein A columns to obtain 2 types of purified anti-immunogen antibodies for each immunogen.

### 2. Preparation of label pad

The purified anti-immunogen antibodies were allowed to covalently bind to red latex particles, suspended in a suspension, and subjected to sonication to thoroughly disperse and suspend latex particles. Thus, an anti-RS virus latex suspension, an anti-adenovirus latex suspension, and an anti-Mycoplasma pneumoniae latex suspension were prepared. These latex suspensions were each applied to a glass fiber with a size of 20 cm × 1 cm, and the resultants were thoroughly dried with warm air to prepare label pads each formed of a dried mixture.

### 3. Preparation of sample application pad

A glass fiber with a size of 2.0 cm × 20 cm was used.

### 4. Preparation of test device

A test device of the same constitution as shown in Fig. 1 was used. On a nitrocellulose membrane cut to a size of 2 cm × 20 cm and backed with an adhesive plastic plate, a solution of the anti-RS virus antibody (an antibody different from the above antibody), a solution of the anti-adenovirus antibody (an antibody different from the above antibody), or a solution of the anti-Mycoplasma pneumoniae antibody was applied in a coating amount of about 1 mm in width at sites 0.8 cm and 1.0 cm from the lower end across the width of 20 cm, followed by thorough drying with warm air to immobilize the antibodies (the detection units). Subsequently, a filter paper with a size of 3 cm × 20 cm was laid on the nitrocellulose membrane to overlap therewith by 5 mm from the upper end of the nitrocellulose membrane to provide an absorption pad unit. Further, the label pad was laid on the nitrocellulose membrane to overlap therewith by 2 mm from the lower end of the nitrocellulose membrane to provide a label unit. Further, the sample application pad was laid on the label pad at a position 7 mm away from the upper end of the label pad to provide a sample application unit. Subsequently, the resultant was cut into strips with a width of 5 mm using a knife to obtain an integrated test device.

### 5. Preparation of specimen-extracting solution

Preparation of a specimen-extracting solution is described in the test examples below.

### Test example 1

Effects of specimen-extracting solution containing L-phenylalanine (L-Phe) for suppressing false positives when detecting influenza virus by immunochromatography 1-1. Preparation of specimen-extracting solution

A specimen-extracting solution (Control 1) was prepared by mixing 50 mM Tris buffer (pH 8.0), 2 (w/v)% polyoxyethylene octyl phenyl ether, and 5 (w/v)% L-arginine. Subsequently, a specimen-extracting solution (Test 1) was prepared by mixing 50 mM Tris buffer (pH 8.0), 1.25 (w/v)% polyoxyethylene alkyl ether, 0.75 (w/v)% polyoxyethylene octyl phenyl ether, 4 (w/v)% glycine ethyl ester, 1.5 (w/v)% L-Phe, and 400 mM sodium bromide.

### 1-2. Test method

The cryopreserved influenza-virus-negative nasal aspirate in which false positives had been observed with the control specimen-extracting solution was thawed and the specimens were collected with the use of cotton swabs. The specimens were suspended and dispersed in the control specimen-extracting solution and in the specimen-extracting solution (Test 1) to prepare the test samples. Subsequently, the resulting test samples were applied dropwise to the test device prepared above, and color intensity of the detection unit was measured 5 minutes later. Color intensity was measured with the use of the red color chart and the blue color chart each on a zero-to-ten scale.

### 1-3. Results of color intensity test

The results are shown in Table 1. Color intensity indicated by a numeral value increases in ascending order from 0, 1+, 2+... 10+, "0" indicates that color development was not observed, and a higher value indicates a higher signal intensity. The results are shown in terms of "color intensity of influenza A/color intensity of influenza B."

While false positives were observed with the use of the control specimen-extracting solution, false positives were suppressed in all specimens with the use of the specimen-extracting solution (Test 1) containing L-phenylalanine.

**[Table 1]**

| Effects of L-Phe-containing specimen-extracting solution for suppressing false positives (immunochromatography) | | | | | |
|---|---|---|---|---|---|
| Specimen No. | Specimen-extracting solution | | Specimen No. | Specimen-extracting solution | |
| | Control 1 | Test 1 | | Control 1 | Test 1 |
| Specimen 1 | 1+/1+ | 0/0 | Specimen 9 | 1+/2+ | 0/0 |
| Specimen 2 | 2+/4+ | 0/0 | Specimen 10 | 3+/4+ | 0/0 |
| Specimen 3 | 2+/2+ | 0/0 | Specimen 11 | 1+/0 | 0/0 |
| Specimen 4 | 3+/4+ | 0/0 | Specimen 12 | 1+/1+ | 0/0 |
| Specimen 5 | 1+/2+ | 0/0 | Specimen 13 | 1+/2+ | 0/0 |
| Specimen 6 | 1+/1+ | 0/0 | Specimen 14 | 2+/1+ | 0/0 |
| Specimen 7 | 1+/2+ | 0/0 | Specimen 15 | 0/1+ | 0/0 |
| Specimen 8 | 1+/1+ | 0/0 | | | |

| | | | | | |
|---|---|---|---|---|---|
| ^{∗}1. The results are shown "color intensity of Flu A/color intensity of Flu B." ^{∗}2. Color intensity indicated by a numeral value increases in ascending order from 1+, 2+... 10+ and a higher value indicates a higher signal intensity. | | | | | |

### Test example 2

### Verification of effects of L-Phe by itself for suppressing false positives

### 2-1. Preparation of specimen-extracting solution

A specimen-extracting solution (Control 2) was prepared by mixing 50 mM Tris buffer (pH 8.0), 1.25 (w/v)% polyoxyethylene alkyl ether, 0.75 (w/v)% polyoxyethylene octyl phenyl ether, 4 (w/v)% glycine ethyl ester, and 400 mM sodium bromide. Subsequently, a specimen-extracting solution (Test 2) was prepared by mixing 50 mM Tris buffer (pH 8.0), 1.25 (w/v)% polyoxyethylene alkyl ether, 0.75 (w/v)% polyoxyethylene octyl phenyl ether, 4 (w/v)% glycine ethyl ester, 400 mM sodium bromide, and 1.5 (w/v)% L-Phe.

### 2-2. Test method

The cryopreserved influenza-virus-negative nasal aspirate in which false positives had been observed with the specimen-extracting solution (Control 1) was thawed and the specimens were collected with the use of cotton swabs. The specimens were suspended and dispersed in the specimen-extracting solution (Control 1) and in the specimen-extracting solution (Test 2) to prepare the test samples. Subsequently, the resulting test samples were applied dropwise to the test device prepared above, and color intensity of the detection unit was measured 5 minutes later. Color intensity was measured with the use of the red color chart and the blue color chart each on a zero-to-ten scale.

### 2-3. Results of color intensity test

The results are shown in Table 2. Color intensity indicated by a numeral value increases in ascending order from 0, 1+, 2+... 10+, "0" indicates that color development was not observed, and a higher value indicates a higher signal intensity. The results are shown in terms of "color intensity of type A/color intensity of type B."

**[Table 2]**

| Specimen No. | Specimen-extracting solution | |
|---|---|---|
| | Control 2 | Test 2 |
| Specimen 16 | 5+/5+ | 0/0 |
| Specimen 17 | 0/1+ | 0/0 |
| Specimen 18 | 1+/0 | 0/0 |
| Specimen 19 | 0/1+ | 0/0 |
| Specimen 20 | 1+/1+ | 0/0 |

| | | |
|---|---|---|
| * 1. The results are shown "color intensity of type A/color intensity of type B." *2. Color intensity indicated by a numeral value increases in ascending order from 1+, 2+...10+ and a higher value indicates a higher signal intensity. | | |

### Test example 3

### Comparison of sensitivity between L-Phe-containing specimen-extracting solution and conventional specimen-extracting solution

### 3-1. Preparation of specimen-extracting solution

A specimen-extracting solution (Control 1) was prepared by mixing 50 mM Tris buffer (pH 8.0), 2 (w/v)% polyoxyethylene octyl phenyl ether, and 5 (w/v)% L-arginine. Subsequently, a specimen-extracting solution (Test 1) was prepared by mixing 50 mM Tris buffer (pH 8.0), 1.25 (w/v)% polyoxyethylene alkyl ether, 0.75 (w/v)% polyoxyethylene octyl phenyl ether, 4 (w/v)% glycine ethyl ester, 1.5 (w/v)% L-Phe, and 150 mM sodium bromide.

### 3-2. Test method

The inactivated influenza A viruses were added to the specimen-extracting solution (Control 1) and the specimen-extracting solution (Test 1) to the final concentrations of 6.8 × 10² PFU/ml, 3.4 × 10² PFU/ml, and 1.7 × 10² PFU/ml, respectively, to prepare samples. Also, the inactivated influenza B viruses were added to the specimen-extracting solutions to the final concentrations of 4.0 × 10² PFU/ml, 2.0 × 10² PFU/ml, and 1.0 × 10² PFU/ml, respectively, to prepare samples. Subsequently, these samples were applied dropwise to the test device prepared above in amounts of 50 µl each, and color intensity of the detection unit was measured 5 minutes later. Color intensity was measured with the use of the red color chart and the blue color chart each on a zero-to-ten scale.

### 3-3. Results of color intensity test

The results are shown in Table 3. Color intensity indicated by a numeral value increases in ascending order from 0, 0.5+, 1+, 2+...10+, "0" indicates that color development was not observed, and a higher value indicates a higher signal intensity. The results are shown in terms of "color intensity of influenza A/color intensity of influenza B."

Sensitivity of the specimen-extracting solution (Control 1) was equivalent to that of the specimen-extracting solution (Test 1). The signal intensity of Test 1 was higher than that of Control 1. The results demonstrate that specificity can be improved without lowering sensitivity with the use of the specimen-extracting solution prepared in the present invention.

**[Table 3]**

| Comparison of sensitivity between L-Phe-containing specimen-extracting solution and conventional specimen-extracting solution | | | | | | |
|---|---|---|---|---|---|---|
| Antigen type | Influenza A virus | | | Influenza B virus | | |
| Antigen level (PFU/ml) | 6.8 × 10² | 3.4 × 10² | 1.7 × 10² | 4.0 × 10 | 2.0 × 10 | 1.0 × 10 |
| Control 1 | 4+/0 | 1+/0 | 0.5+/0 | 0/3+ | 0/1+ | 0/0.5+ |
| Test 1 | 5+/0 | 2+/0 | 0.5+/0 | 0/5+ | 0/2+ | 0/1+ |

### Test example 4

### Effects of specimen-extracting solution containing L-Phe analog for suppressing false positives

### 4-1. Preparation of specimen-extracting solution

A control specimen-extracting solution (without additives) was prepared by mixing 50 mM Tris buffer (pH 8.0), 2 (w/v)% polyoxyethylene octyl phenyl ether, and 2 (w/v)% L-arginine. Subsequently, a specimen-extracting solution to be subjected to the test was prepared by mixing 50 mM Tris buffer (pH 8.0), 2 (w/v)% polyoxyethylene octyl phenyl ether, 2 (w/v)% L-arginine, and one of the compounds shown in Table 4 (figure in parentheses: final concentration).

**[Table 4]**

| List of additives to specimen-extracting solution and abbreviation | |
|---|---|
| Additives (final concentration) | Abbreviation |
| L-Phenylalanine (2.0%) | L-Phe |
| D-Phenylalanine (2.0%) | D-Phe |
| Aspartame (1.0%) | - |
| Hippuric acid (2.0%) | - |
| ± Mandelic acid (2.0%) | - |
| L-Tryptophan (1.0%) | L-Trp |
| L-Phenylalanine methyl ester (2.0%) | L-PheME |
| N-Phenylglycine (2.0%) | N-PG |
| DL-2-Phenylglycine (0.1%) | 2-PG |
| Sodium benzoate (2.0%) | - |
| 3-Phenylpropionic acid (1.5%) | 3PPA |
| N-(m-toluoyl)glycine (1.5%) | NmTG |
| Acetylsalicylic acid (1.5%) | AcSA |

### 4-2. Test method

The cryopreserved influenza-virus-negative nasal aspirate in which false positives had been observed with the control specimen-extracting solution was thawed and the specimens were collected with the use of cotton swabs. These specimens were suspended and dispersed in the control specimen-extracting solution and in the test specimen-extracting solutions to prepare the test samples. Subsequently, the resulting test samples were applied dropwise to the test device prepared above, and color intensity of the detection unit was measured 5 minutes later. Color intensity was measured with the use of the red color chart and the blue color chart each on a zero-to-ten scale.

### 4-3. Results of color intensity test

The results are shown in Table 5, Table 6, and Table 7. In the tables, the effects are shown in terms of "influenza A/influenza B," "Good" indicates an additive exerting effects of suppressing false positives, and "Poor" indicates an additive exerting no effects of suppressing false positives.

While the results vary among specimens, false-positive reaction that could not be sufficiently suppressed with L-arginine were suppressed with the additives shown in Table 4.

**[Table 5]**

| Effects of specimen-extracting solution containing L-Phe analog for suppressing false positives | | | | | | | |
|---|---|---|---|---|---|---|---|
| Specimen No. | Additive type | | | | | | |
| | No additive | L-Phe | D-Phe | Aspartame | Hippuric acid | ±Mandelic acid | L-Trp |
| Specimen 21 | Poor/Poor | Good/Good | Good/Good | Good/Good | Good/Good | Good/Good | Good/Good |
| Specimen 22 | Poor/Poor | Poor/Poor | Poor/Poor | Good/Poor | Good/Good | Poor/Poor | Poor/Poor |
| Specimen 23 | Poor/Poor | Poor/Poor | Poor/Poor | Poor/Poor | Good/Good | Good/Good | Good/Good |
| Specimen 24 | Poor/Poor | Good/Good | Poor/Poor | Poor/Poor | Good/Good | Good/Good | Poor/Poor |
| Specimen 25 | Poor/Poor | Poor/Poor | Good/Good | Poor/Poor | Good/Good | Poor/Poor | Poor/Good |
| Specimen 26 | Poor/Poor | Good/Good | Good/Good | Good/Good | Good/Good | Good/Good | Good/Good |
| Specimen 27 | Poor/Poor | Good/Poor | Good/Good | Good/Good | Good/Good | Good/Good | Good/Poor |
| Specimen 28 | Poor/Poor | Good/Good | Good/Good | Good/Good | Good/Good | Good/Good | Good/Good |
| Specimen 29 | Poor/Poor | Good/Good | Poor/Poor | Poor/Poor | Good/Good | Good/Good | Good/Poor |
| Specimen 30 | Poor/Poor | Good/Good | Good/Good | Good/Good | Good/Good | Good/Good | Good/Good |

**[Table 6]**

| Specimen No. | Additive type | | | | |
|---|---|---|---|---|---|
| | No additive | L-PheME | N-PG | 2-PG | Sodium benzoate |
| Specimen 31 | Poor/Poor | Good/Good | Good/Good | Good/Good | Good/Good |
| Specimen 32 | Poor/Poor | Poor/Poor | Poor/Poor | Good/Good | Good/Good |
| Specimen 33 | Poor/Poor | Good/Good | Poor/Poor | Good/Good | Good/Good |
| Specimen 34 | Poor/Poor | Poor/Poor | Poor/Poor | Good/Good | Good/Good |
| Specimen 35 | Poor/Poor | Good/Good | Good/Good | Good/Good | Good/Good |
| Specimen 36 | Poor/Poor | Poor/Poor | Poor/Poor | Good/Good | Good/Good |

**[Table 7]**

| Specimen No. | Additive type | | | |
|---|---|---|---|---|
| | No additive | 3PPA | NmTG | AcSA |
| Specimen 37 | Poor/Poor | Poor/Good | Poor/Good | Poor/Good |
| Specimen 38 | Poor/Poor | Good/Good | Good/Good | Poor/Poor |
| Specimen 39 | Poor/Poor | Good/Good | Good/Good | Good/Good |
| Specimen 40 | Poor/Poor | Good/Good | Poor/Poor | Poor/Good |
| Specimen 41 | Poor/Poor | Good/Good | Good/Good | Poor/Poor |
| Specimen 42 | Poor/Poor | Poor/Good | Good/Good | Good/Good |
| Specimen 43 | Poor/Poor | Good/Good | Good/Good | Poor/Poor |
| Specimen 44 | Poor/Poor | Good/Good | Good/Good | Good/Good |
| Specimen 45 | Poor/Poor | Good/Good | Good/Good | Good/Good |
| Specimen 46 | Poor/Poor | Good/Good | Good/Good | Good/Good |

### Test Example 5

### Effects of L-Phe-containing specimen-extracting solution for suppressing false positives when detecting RS virus, adenovirus, and Mycoplasma pneumoniae by immuno chromatography

### 5-1. Preparation of specimen-extracting solution

A specimen-extracting solution (Control 1) was prepared by mixing 50 mM Tris buffer (pH 8.0), 2 (w/v)% polyoxyethylene octyl phenyl ether, and 5 (w/v)% L-arginine. Subsequently, a specimen-extracting solution (Test 4) was prepared by mixing 50 mM Tris buffer (pH 8.0), 1.25 (w/v)% polyoxyethylene alkyl ether, 0.75 (w/v)% polyoxyethylene octyl phenyl ether, 4 (w/v)% glycine ethyl ester, 1.5 (w/v)% L-Phe, and 150 mM sodium bromide.

### 5-2. Test method

The cryopreserved RS virus-, adenovirus-, and Mycoplasma pneumoniaenegative nasal aspirates in which false positives had been observed with the control specimen-extracting solution were thawed and the specimens were collected with the use of cotton swabs. The specimens were suspended and dispersed in the control specimen-extracting solution and in the specimen-extracting solution (Test 4) to prepare the test samples. Subsequently, the resulting test samples were applied dropwise to the test device prepared above. The RS virus sample and the adenovirus sample were subjected to measurement of color intensity of the detection unit 5 minutes later, and the Mycoplasma pneumoniae sample was subjected to the measurement 15 minutes later. Color intensity was measured with the use of the red color chart and the blue color chart each on a zero-to-ten scale.

### 5-3. Results of color intensity test

The results of the RS virus sample are shown in Table 8, those of the adenovirus sample are shown in Table 9, and those of the Mycoplasma pneumoniae sample are shown in Table 10. Color intensity indicated by a numeral value increases in ascending order from 0, 1+, 2+...10+, "0" indicates that color development was not observed, and a higher value indicates a higher signal intensity.

In all of the above results, false positives were observed with the control specimen-extracting solution; however, false positives were suppressed in all the specimens with the use of the L-phenylalanine-containing specimen-extracting solution (Test 4).

**[Table 8]**

| Effects of L-Phe-containing specimen-extracting solution for suppressing false positives when detecting RS viruses | | |
|---|---|---|
| Specimen No. | Control 1 | Test 4 |
| Specimen A1 | 1+ | 0 |
| Specimen A2 | 2+ | 0 |

**[Table 9]**

| Effects of L-Phe-containing specimen-extracting solution for suppressing false positives when detecting adenoviruses | | |
|---|---|---|
| Specimen No. | Control 1 | Test 4 |
| Specimen A3 | 5+ | 0 |
| Specimen A4 | 1+ | 0 |
| Specimen A5 | 2+ | 0 |
| Specimen A6 | 1+ | 0 |
| Specimen A7 | 6+ | 0 |
| Specimen A8 | 1+ | 0 |

**[Table 10]**

| Effects of L-Phe-containing specimen-extracting solution for suppressing false positives when detecting Mycoplasma pneumoniae | | |
|---|---|---|
| Specimen No. | Control 1 | Test 4 |
| Specimen A9 | 1+ | 0 |

### Industrial Applicability

The method of the present invention can be used to accurately detect various substances.

### Description of Numeral References

1: Nitrocellulose membrane
2: Label part
3: Detection part
4: Sample application part
5: Absorption pad part
6: Plastic plate

All publications, patents, and patent applications cited herein are incorporated herein by reference in their entirety.

## Claims

1. A test reagent for detecting a target substance in a specimen utilizing the antigen-antibody reactions or the binding reactions between substances interactive with each other, which comprises a specimen-extracting solution containing a non-specific-reaction-suppressing component for suppressing false positives, which is a water-soluble compound with a molecular weight of 6,000 Da or lower containing a phenyl group, a benzyl group, a tolyl group, or a xylyl group, further containing a carboxyl group, a methoxycarboxyl group, or an ethoxycarboxyl group, and optionally containing a hydroxyl group.

2. The test reagent according to Claim 1, which is a test reagent for immunochromatography or a test device for immunochromatography comprising a specimen-extracting solution containing a non-specific-reaction-suppressing component for suppressing false positives, which is a water-soluble compound with a molecular weight of 6,000 Da or lower containing a phenyl group, a benzyl group, a tolyl group, or a xylyl group, further containing a carboxyl group, a methoxycarboxyl group, or an ethoxycarboxyl group, and optionally containing a hydroxyl group.

3. The test reagent according to Claim 1 or 2, which is a test device for immunochromatography comprising a site impregnated with a specimen-extracting solution containing a non-specific-reaction-suppressing component for suppressing false positives, which is a water-soluble compound with a molecular weight of 6,000 Da or lower containing a phenyl group, a benzyl group, a tolyl group, or a xylyl group, further containing a carboxyl group, a methoxycarboxyl group, or an ethoxycarboxyl group, and optionally containing a hydroxyl group.

4. The test reagent according to any one of Claims 1 to 3, wherein the specimen-extracting solution contains 0.1 to 10 (w/v)% of a non-specific-reaction-suppressing component for suppressing false positives.

5. The test reagent according to any one of Claims 1 to 4, which is a compound or tryptophan represented by any of Formulae (I) to (V) below: 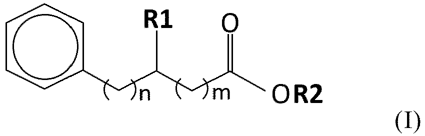 in Formula (I), R1 represents H, OH, =O, NH₂, COOH, NH-CO-CNH₂-C-COOH, or CH₃; R2 represents H, CH₃, C₂H₅, Li, Na, K, Rb, Cs, or Fr; n is 0 or 1; and m is 0, 1, 2, 3, or 4; 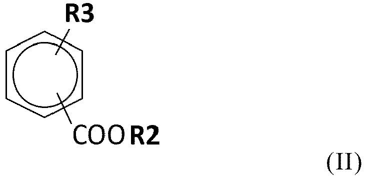 in Formula (II), R2 represents H, CH₃, C₂H₅, Li, Na, K, Rb, Cs, or Fr; R3 represents COOH, COOLi, COONa, COOK, COORb, COOCs, COOFr, COOCH ₃, COOC 2 H₅, OCOH, or CH₃; and R3 and COOR2 are each in the ortho-, meta-, or para-position in a benzene ring; 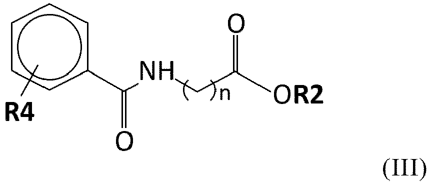 in Formula (III), R2 represents H, CH₃, C₂H₅, Li, Na, K, Rb, Cs, or Fr; R4 represents H or CH₃; and n is 0 or 1; 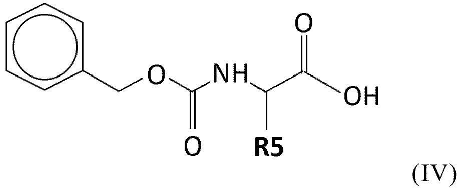 in Formula (IV), R5 is a side chain of glycine, alanine, valine, leucine, isoleucine, serine, threonine, cysteine, methionine, asparagine, glutamine, phenylalanine, tyrosine, tryptophan, glutamic acid, aspartic acid, arginine, lysine, or histidine; and 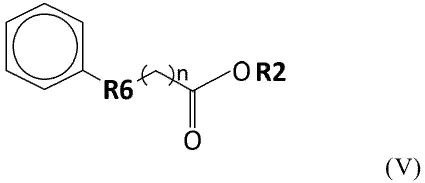 in Formula (V), R2 represents H, CH₃, C₂H₅, Li, Na, K, Rb, Cs, or Fr; R6 represents NH or O; and n is 0, 1, 2, 3, or 4.

6. The test reagent according to any one of Claims 1 to 5, wherein the water-soluble compound with a molecular weight of 6,000 Da or lower containing a phenyl group, a benzyl group, a tolyl group, or a xylyl group, further containing a carboxyl group, a methoxycarboxyl group, or an ethoxycarboxyl group, and optionally containing a hydroxyl group is a compound selected from the group consisting of aspartame, phenylalanine, phenylalanine methyl ester, mandelic acid, 2-phenylpropionic acid, 3-phenylpropionic acid, phenylglycine, phenylglycine methyl ester, phenylglycine ethyl ester, phenyllactic acid, phenylpyruvic acid, benzoic acid, phthalic acid, acetylsalicylic acid, hippuric acid, N-toluoylglycine, N-carbobenzyloxyamino acid, N-phenylglycine, phenoxyacetic acid, tryptophan, metal salts of the compounds, and optical isomers, geometric isomers, constitutional isomers, stereoisomers, and positional isomers of the compounds.

7. The test reagent according to any one of Claims 1 to 6, wherein the specimen-extracting solution is further containing an amino acid or an amino acid derivative selected from the group consisting of arginine, lysine, arginine ethyl ester, arginine methyl ester, glycine ethyl ester, glycine methyl ester, and optical isomers, geometric isomers, constitutional isomers, and stereoisomers of the compounds.

8. The test reagent according to any one of Claims 1 to 7, wherein the specimen-extracting solution is further containing a halide selected from the group consisting of lithium chloride, sodium chloride, potassium chloride, sodium bromide, potassium bromide, sodium iodide, and potassium iodide.

9. A method for detecting a target substance selected from the group consisting of a virus antigen, a bacterial antigen, and a protein antigen in a specimen selected from the group consisting of a pharyngeal swab specimen, a nasal swab specimen, a nasal aspirate specimen, a pharyngeal wash specimen, a nasal wash specimen, a nasal secretion specimen collected by nose blowing, a saliva specimen, a serum specimen, a plasma specimen, a whole blood specimen, a fecal specimen, a fecal suspension specimen, and an urine specimen utilizing the antigen-antibody reactions or the reactions between substances interactive with each other in a specimen-extracting solution while suppressing the false positives, wherein the specimen is brought into contact with a non-specific-reaction-suppressing component for suppressing false positives, which is a water-soluble compound with a molecular weight of 6,000 Da or lower containing a phenyl group, a benzyl group, a tolyl group, or a xylyl group, further containing a carboxyl group, a methoxycarboxyl group, or an ethoxycarboxyl group, and optionally containing a hydroxyl group, in advance.

10. The method according to Claim 9, wherein the method for detecting a target substance is an immunochromatography method comprising introducing a specimen into a specimen-extracting solution containing a non-specific-reaction-suppressing component for suppressing false positives, which is a water-soluble compound with a molecular weight of 6,000 Da or lower containing a phenyl group, a benzyl group, a tolyl group, or a xylyl group, further containing a carboxyl group, a methoxycarboxyl group, or an ethoxycarboxyl group, and optionally containing a hydroxyl group, and applying the specimen-extracting solution to a test device for immunochromatography.

11. The method according to Claim 9, wherein the method for detecting a target substance is an immunochromatography method comprising applying a specimen to a test device for immunochromatography comprising a site impregnated with a specimen-extracting solution containing a non-specific-reaction-suppressing component for suppressing false positives, which is a water-soluble compound with a molecular weight of 6,000 Da or lower containing a phenyl group, a benzyl group, a tolyl group, or a xylyl group, further containing a carboxyl group, a methoxycarboxyl group, or an ethoxycarboxyl group, and optionally containing a hydroxyl group.

12. The method according to any one of Claims 9 to 11, wherein the specimen-extracting solution contains 0.1 to 10 (w/v)% of a non-specific-reaction-suppressing component for suppressing false positives.

13. The method according to any one of Claims 9 to 12, wherein the water-soluble compound with a molecular weight of 6,000 Da or lower containing a phenyl group, a benzyl group, a tolyl group, or a xylyl group, further containing a carboxyl group, a methoxycarboxyl group, or an ethoxycarboxyl group, and optionally containing a hydroxyl group is a compound or tryptophan represented by any of Formulae (I) to (V) below: 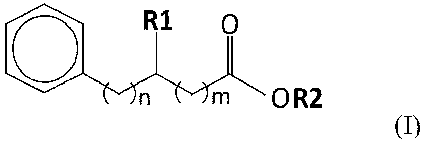 in Formula (I), R1 represents H, OH, =O, NH₂, COOH, NH-CO-CNH₂-C-COOH, or CH₃; R2 represents H, CH₃, C₂H₅, Li, Na, K, Rb, Cs, or Fr; n is 0 or 1; and m is 0, 1, 2, 3, or 4; 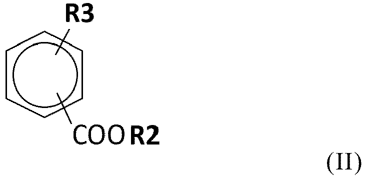 in Formula (II), R2 represents H, CH₃, C₂H₅, Li, Na, K, Rb, Cs, or Fr; R3 represents COOH, COOLi, COONa, COOK, COORb, COOCs, COOFr, COOCH ₃, COOC₂H₅, OCOH, or CH₃; and R3 and COOR2 are each in the ortho-, meta-, or para-position in a benzene ring; 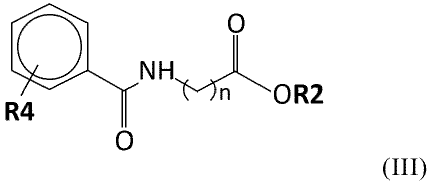 in Formula (III), R2 represents H, CH₃, C₂H₅, Li, Na, K, Rb, Cs, or Fr; R4 represents H or CH₃; and n is 0 or 1; 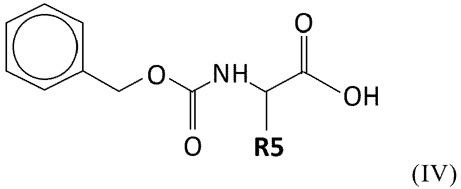 in Formula (IV), R5 is a side chain of glycine, alanine, valine, leucine, isoleucine, serine, threonine, cysteine, methionine, asparagine, glutamine, phenylalanine, tyrosine, tryptophan, glutamic acid, aspartic acid, arginine, lysine, or histidine; and 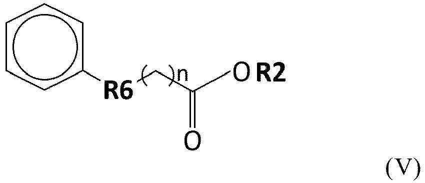 in Formula (V), R2 represents H, CH₃, C₂H₅, Li, Na, K, Rb, Cs, or Fr; R6 represents NH or O; and n is 0, 1, 2, 3, or 4.

14. The method according to any one of Claims 9 to 13, wherein the water-soluble compound with a molecular weight of 6,000 Da or lower containing a phenyl group, a benzyl group, a tolyl group, or a xylyl group, further containing a carboxyl group, a methoxycarboxyl group, or an ethoxycarboxyl group, and optionally containing a hydroxyl group is a compound selected from the group consisting of aspartame, phenylalanine, phenylalanine methyl ester, mandelic acid, 2-phenylpropionic acid, 3-phenylpropionic acid, phenylglycine, phenylglycine methyl ester, phenylglycine ethyl ester, phenyllactic acid, phenylpyruvic acid, benzoic acid, phthalic acid, acetylsalicylic acid, hippuric acid, N-toluoylglycine, N-carbobenzyloxyamino acid, N-phenylglycine, phenoxyacetic acid, tryptophan, metal salts of the compounds, and optical isomers, geometric isomers, constitutional isomers, stereoisomers, and positional isomers of the compounds.

15. The method according to any one of Claims 9 to 14, wherein the specimen-extracting solution is further supplemented with an amino acid or an amino acid derivative selected from the group consisting of arginine, lysine, arginine ethyl ester, arginine methyl ester, glycine ethyl ester, glycine methyl ester, and optical isomers, geometric isomers, constitutional isomers, and stereoisomers of the compounds.

16. The method according to any one of Claims 9 to 15, wherein the specimen-extracting solution is further supplemented with a halide selected from the group consisting of lithium chloride, sodium chloride, potassium chloride, sodium bromide, potassium bromide, sodium iodide, and potassium iodide.
